# EUROPEAN PATENT SPECIFICATION

(11) **EP 2 813 142 B1**
(45) Date of publication and mention of the grant of the patent: **23.05.2018**
(21) Application number: 13746062.2
(22) Date of filing: 08.02.2013
(51) Int. Cl.: A01H 5/00, C07K 7/04, C07K 14/00, C07K 14/415

(54) **METHOD FOR INTRODUCING FLORIGEN**
VERFAHREN ZUM EINBRINGEN VON FLORIGEN
PROCÉDÉ POUR L'INTRODUCTION DE FLORIGÈNE

(30) Priority: 08.02.2012 JP 2012024958
(43) Date of publication of application: 17.12.2014
(73) Proprietor: National University Corporation Nara Institute of Science and Technology, Ikoma-shi, Nara 630-0192 (JP)
(72) Inventor: TSUJI, Hiroyuki, Ikoma-shi Nara 630-0192 (JP); SHIMAMOTO, Ko, Ikoma-shi Nara 630-0192 (JP); TAOKA, Ken-ichiro, Ikoma-shi Nara 630-0192 (JP); WASHIDA, Haruhiko, Ikoma-shi Nara 630-0192 (JP)
(74) Representative: Krauss, Jan
(86) International application number: PCT/JP2013/053043
(87) International publication number: WO 2013/118863

(56) References cited:
- WO-A1-02/42475
- WO-A1-2011/059051
- JP-A- 2010 532 159
- CHIN CHIN YEOH ET AL: "Developing a method for customized induction of flowering", BMC BIOTECHNOLOGY, BIOMED CENTRAL LTD. LONDON, GB, vol. 11, no. 1, 11 April 2011 (2011-04-11) , page 36, XP021096717, ISSN: 1472-6750, DOI: 10.1186/1472-6750-11-36
- CHANG M ET AL.: 'Cellular internalization of fluorescent proteins via arginine-rich intracellular delivery peptide in plant cells' PLANT CELL PHYSIOL. vol. 46, no. 3, 2005, pages 482 - 8, XP008126698
- DOU D ET AL.: 'RXLR-mediated entry of Phytophthora sojae effector Avrlb into soybean cells does not require pathogen-encoded machinery' PLANT CELL. vol. 20, no. 7, 2008, pages 1930 - 47, XP055032784
- HARUHIKO WASHIDA ET AL.: 'DIRECT PROTEIN INTRODUCTION INTO PLANT CELLS WITHOUT GENE TRANSFER' BREEDING RESEARCH vol. 12, no. 2, 2010, page 215, XP008174728
- TAOKA,K. ET AL.: '14-3-3 proteins act as intracellular receptors for rice Hd3a florigen' NATURE vol. 476, no. 7360, 2011, LOND, pages 332 - 335, XP055159540
- PURWESTRI YA ET AL.: 'The 14-3-3 protein GF14c acts as a negative regulator of flowering in rice by interacting with the florigen Hd3a' PLANT CELL PHYSIOL. vol. 50, no. 3, 2009, pages 429 - 38, XP002716928
- SEIKAGAKU JITEN KABUSHIKI KAISHA TOKYO KAGAKU DOJIN 1998, page 415, XP008174945
- HARUHIKO WASHIDA ET AL.: 'DIRECT RICE FLORIGEN HD3A PROTEIN TRANSDUCTION INTO PLANT CELLS USING CELL PENETRATING PEPTIDE' BREEDING RESEARCH vol. 14, 29 March 2012, page 144, XP008174727

### SEQUENCE LISTING

<110> NATIONAL UNIVERSITY CORPORATION NARA INSTITUTE OF SCIENCE
<120> Method for introducing florigen
<130> GP12-1025PCT
<150> JP2012-024955
   <151> 2012-02-08
<160> 22
<170> **PatentIn** version 3.5
<210> 1
   <211> 179
   <212> PRT
   <213> Oryza sativa
<400> 1
   Met Ala Gly Ser Gly Arg Asp Arg Asp Pro Leu Val Val Gly Arg Val
   1               5                   10                  15
   Val Gly Asp Val Leu Asp Ala Phe Val Arg Ser Thr Asn Leu Lys Val
               20                  25                  30
   Thr Tyr Gly Ser Lys Thr Val Ser Asn Gly Cys Glu Leu Lys Pro Ser
           35                  40                  45
   Met Val Thr His Gln Pro Arg Val Glu Val Gly Gly Asn Asp Met Arg
       50                  55                  60
   Thr Phe Tyr Thr Leu Val Met Val Asp Pro Asp Ala Pro Ser Pro Ser
   65                  70                  75                  80
   Asp Pro Asn Leu Arg Glu Tyr Leu His Trp Leu Val Thr Asp Ile Pro
                   85                  90                  95
   Gly Thr Thr Ala Ala Ser Phe Gly Gln Glu Val Met Cys Tyr Glu Ser
               100                 105                 110
   Pro Arg Pro Thr Met Gly Ile His Arg Leu Val Phe Val Leu Phe Gln
           115                 120                 125
   Gln Leu Gly Arg Gln Thr Val Tyr Ala Pro Gly Trp Arg Gln Asn Phe
       130                 135                 140
   Asn Thr Lys Asp Phe Ala Glu Leu Tyr Asn Leu Gly Ser Pro Val Ala
   145                 150                 155                 160
   Ala Val Tyr Phe Asn Cys Gln Arg Glu Ala Gly Ser Gly Gly Arg Arg
                     165                 170                 175
   Val Tyr Pro
<210> 2
   <211> 11
   <212> **PRT**
   <213> **Artificial** Sequence
<220>
   <223> Membrane-permeable peptide Tat
<400> 2
   Tyr Gly Arg Lys Lys Arg Arg Gln Arg Arg Arg
   1               5                   10
<210> 3
   <211> 15
   <212> **PRT**
   <213> **Artificial** Sequence
<220>
   <223> Membrane-permeable peptide **FHVcoat**
<400> 3
   Arg Arg Arg Arg Asn Arg Thr Arg Arg Asn Arg Arg Arg Val Arg
   1               5                   10                  15
<210> 4
   <211> 21
   <212> **PRT**
   <213> **Artificial** Sequence
<220>
   <223> Membrane-permeable peptide Pep-1
<400> 4
   Lys Glu Thr Trp Trp Glu Thr Trp Trp Thr Glu Trp Ser Gln Pro Lys
   1               5                   10                  15
   Lys Lys Arg Lys Val
               20
<210> 5
   <211> 16
   <212> PRT
   <213> Artificial Sequence
<220>
   <223> Membrane-permeable peptide Penetratin
<400> 5
   Arg Gln Ile Lys Ile Trp Phe Gln Asn Arg Arg Met Lys Trp Lys Lys
   1               5                   10                  15
<210> 6
   <211> 27
   <212> PRT
   <213> Artificial Sequence
<220>
   <223> Membrane-permeable peptide Transportan
<400> 6
   Gly Trp Thr Leu Asn Ser Ala Gly Tyr Leu Leu Gly Lys Ile Asn Leu
   1               5                   10                  15
   Lys Ala Leu Ala Ala Leu Ala Lys Lys Ile Leu
               20                  25
<210> 7
   <211> 9
   <212> PRT
   <213> Artificial Sequence
<220>
   <223> Membrane-permeable peptide R9
<400> 7
   Arg Arg Arg Arg Arg Arg Arg Arg Arg
   1               5
<210> 8
   <211> 30
   <212> PRT
   <213> Artificial Sequence
<220>
   <223> Membrane-permeable peptide KALA
<400> 8
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 9
   <211> 45
   <212> PRT
   <213> Artificial Sequence
<220>
   <223> Membrane-permeable peptide Avr1b
<400> 9
   Thr Glu Tyr Ser Asp Glu Thr Asn Ile Ala Met Val Glu Ser Pro Asp
   1 5 10 15
   Leu Val Arg Arg Ser Leu Arg Asn Gly Asp Ile Ala Gly Gly Arg Phe
   20 25 30
   Leu Arg Ala His Glu Glu Asp Asp Ala Gly Glu Arg Thr
   35 40 45
<210> 10
   <211> 16
   <212> PRT
   <213> Artificial Sequence
<220>
   <223> Membrane-permeable peptide p-Antp
<400> 10
   Arg Gln Ile Lys Ile Trp Phe Gln Asn Arg Arg Met Lys Trp Lys Lys
   1 5 10 15
<210> 11
   <211> 17
   <212> PRT
   <213> Artificial Sequence
<220>
   <223> Membrane-permeable peptide gp41
<400> 11
   Gly Ala Leu Phe Leu Gly Phe Leu Gly Ala Ala Gly Ser Thr Met Gly
   1 5 10 15
   Ala
<210> 12
   <211> 7
   <212> PRT
   <213> Artificial Sequence
<220>
   <223> Membrane-permeable peptide SV40NLS
<400> 12
   Pro Lys Lys Lys Arg Lys Val
   1 5
<210> 13
   <211> 34
   <212> PRT
   <213> Artificial Sequence
<220>
   <223> Membrane-permeable peptide VP22
<400> 13
   Asp Ala Ala Thr Ala Thr Arg Gly Arg Ser Ala Ala Ser Arg Pro Thr
   1               5                   10                  15
   Glu Arg Pro Arg Ala Pro Ala Arg Ser Ala Ser Arg Pro Arg Arg Pro
               20                  25                  30
   Val Asp
<210> 14
   <211> 15
   <212> PRT
   <213> Artificial Sequence
<220>
   <223> Membrane-permeable peptide Integrinbeta3
<400> 14
   Val Thr Val Leu Ala Leu Gly Ala Leu Ala Gly Val Gly Val Gly
   1               5                   10                  15
<210> 15
   <211> 30
   <212> PRT
   <213> Artificial Sequence
<220>
   <223> Membrane-permeable peptide InfluenzaHA-2
<400> 15
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 16
   <211> 21
   <212> PRT
   <213> Artificial Sequence
<220>
   <223> Membrane-permeable peptide BMV-gag
<400> 16
   Lys Met Thr Arg Ala Gln Arg Arg Ala Ala Ala Arg Arg Asn Arg Trp
   1 5 10 15
   Thr Ala Arg Gly Cys
   20
<210> 17
   <211> 13
   <212> PRT
   <213> Artificial Sequence
<220>
   <223> Membrane-permeable peptide HTLV-II Rex
<400> 17
   Thr Arg Arg Gln Arg Thr Arg Arg Ala Arg Arg Asn Arg
   1 5 10
<210> 18
   <211> 9
   <212> PRT
   <213> Artificial Sequence
<220>
   <223> Membrane-permeable peptide R/W
<400> 18
   Arg Arg Trp Trp Arg Arg Trp Arg Arg
   1 5
<210> 19
   <211> 28
   <212> PRT
   <213> Artificial Sequence
<220>
   <223> Membrane-permeable peptide Human c Fos
<400> 19
   Lys Arg Arg Ile Arg Arg Glu Arg Asn Lys Met Ala Ala Ala Lys Ser
   1 5 10 15
   Arg Asn Arg Arg Arg Glu Leu Thr Asp Thr Gly Cys
   20 25
<210> 20
   <211> 30
   <212> PRT
   <213> Artificial Sequence
<220>
   <223> Membrane-permeable peptide Human c Jun
<400> 20
   Arg Ile Lys Ala Glu Arg Lys Arg Met Arg Asn Arg Ile Ala Ala Ser
   1               5                   10                  15
   Lys Ser Arg Lys Arg Lys Leu Glu Arg Ile Ala Arg Gly Cys
               20                  25                  30
<210> 21
   <211> 24
   <212> PRT
   <213> Artificial Sequence
<220>
   <223> Membrane-permeable peptide Yeast GCN 4
<400> 21
   Lys Arg Ala Arg Asn Thr Glu Ala Ala Arg Arg Ser Arg Ala Arg Lys
   1               5                   10                  15
   Leu Gln Arg Met Lys Gln Gly Cys
               20
<210> 22
   <211> 17
   <212> PRT
   <213> Artificial Sequence
<220>
   <223> Membrane-permeable peptide Rev
<400> 22
   Thr Arg Gln Ala Arg Arg Asn Arg Arg Arg Arg Trp Arg Glu Arg Gln
   1               5                   10                  15
   Arg

## Description

### Technical Field

The present invention relates to a method for promoting flowering of a plant according to the appended set of claims.

### Background Art

In order to enhance competitiveness in agricultural production such as food production, production of chemical substances, or biomass production, a technology for controlling flowering of plants is essential. The major technologies for controlling flowering involve those based on an environmental control or a genetic modification. However, the flowering time regulation based on the environmental control cannot be applied universally to various crops or plant species that show lower responsiveness to environmental changes (for example, day length or temperature) or larger-size plant species. Further, in the control of the flowering based on the genetic modification, various crops have been improved by crossing cultivars or genetic engineering technique, but the technology has problems in that: it takes a long time (10 years or more) to create a desirable breed variety; high-quality genetic resources are insufficient; linkage of undesirable characteristics is hard to be avoided; and in some plant species genetic transformation is still difficult to produce transgenic crop.

A florigen is proposed as a universal flowering-determining molecule in plants, and in recent years, the actual molecule of the florigen has been found to be a protein. The florigen is synthesized in a leaf, moves for a long distance up to shoot apices in a plant body, and initiates formation of a flower bud when the florigen reaches the shoot apices. The florigen was considered to be a low-molecular-weight compound when the presence of the florigen was first proposed, and hence it was considered that formation of a flower bud could be induced by treating a plant with the florigen. However, in fact, the florigen is a spherical protein having a molecular weight of about 22,000 and cannot permeate a cell membrane because of its size. Therefore, it is considered that, even if the florigen itself is used for treatment of a plant, the effect cannot be exhibited.

In order to control the flowering of a plant using the florigen, genes coding for the florigen have been isolated (Patent Literature 1, Non Patent Literature 1). The following uses of the genes coding for the florigen have been reported: ear emergence was promoted in rice transformed with the gene coding for the florigen as compared to untransformed rice (Patent Literature 1); and the timing of the flowering was controlled by introducing a mutation into Hd3a, which is one of the genes coding for the florigen (Non Patent Literature 2) . In addition, Non Patent Literature 3 discloses that delay of the flowering was observed in a plant transformed with a homolog of TFL1 gene, which is considered as an antagonistic factor against the florigen. Patent Literature 3 reports that CiFT2 protein was expressed using a citrus unshiu-derived gene having high homology to *Arabidopsis* FT gene, and that the CiFT2 protein was injected into the xylem obtained by cutting a branch of hardy orange. However, in all Examples of Patent Literature 3, it is clearly stated that there is no fact that the treatment with the CiFT2 protein has promoted flower setting. That is, Patent Literature 3 clearly states that, even in a plant not treated with the CiFT2 protein, the flower setting was observed as is the case with the plant treated with the CiFT2 protein (paragraphs 0128 and 0139 in Patent Literature 3). In addition, Patent Literature 3 does not confirm or disclose whether the CiFT2 protein can act as the florigen or not, and whether the CiFT2 protein has been appropriately introduced into a plant body or not.

There have been reported methods of introducing a protein such as a fluorescent protein directly into animal cells or plant cells. One of the methods is a technology using a cell-penetrating peptide (Patent Literature 2, Non Patent Literatures 4 and 5). Non Patent Literature 4 discloses that a fluorescent protein has been introduced into cultured plant cells using a cell-penetrating peptide. Patent Literature 2 discloses that a fluorescent protein or the like has been introduced into various tissues of wheat and the like using a cell-penetrating peptide, and Non Patent Literature 5 discloses that a fluorescent protein has been introduced into the root of onion using a cell-penetrating peptide. However, there is no report that a functional protein specific to a plant such as the florigen has been introduced directly into a tissue of a plant using a cell-penetrating peptide, and that the protein introduced has acted in the plant body.

WO 2011/059051 discloses flowering inducing proteins and their use in inducing flowering in a plant.

### Citation List

### Patent Literature

[PTL 1] WO 2002/042475 A1
[PTL 2] JP 2010-532159 A
[PTL 3] WO 2011/059051 A1

### Non Patent Literature

[NPL 1] Tamaki S et al. Science 2007 May 18; 316 (5827) : 1033-1036.
[NPL 2] Taoka K.-I. et al Nature 2011 476:332-335.
[NPL 3] Nakagawa M et al. Plant J. 2002 29:743-750.
[NPL 4] Zonin et al. Plant Cell Physiol. 52:2225-2235
[NPL 5] Wang HY et al. Biochemical and Biophysical Research Communications 346 (2006) 758-767

### Summary of Invention

### Technical Problem

An object of the present invention is to provide a method of introducing a florigen into a cell of a shoot apical tissue of a plant.

### Solution to Problem

The inventors of the present invention have made intensive studies to accomplish the object, and as a result, have found that a cell-penetrating peptide can be used to introduce a protein of a florigen that is a universal flowering promoting factor directly into a shoot apical tissue where flowering occurs, and that the florigen can promote flowering, thus completing the present invention.

That is, the present invention is defined by the appended set of claims.

### Advantageous Effects of Invention

According to the present invention, it is possible to introduce the florigen directly into the shoot apical tissue where flowering occurs to achieve the flowering-promoting function of the florigen in the shoot apical tissue. According to the method of the present invention, the florigen can be introduced at efficiency as high as 250 times that in a related-art method. The introduction method of the present invention can be applied to a plant species having difficulty in undergoing hybridization breeding or genetic recombination and can be universally used for many plant species. In addition, the size of a plant species into which the florigen is to be introduced is not limited, and the introduction method can be applied outdoors and indoors. Further, the introduction method of the present invention is useful because the method does not require a long-term technical training unlike genetic recombination or the like and can be carried out easily by any person.

### Brief Description of Drawings

FIGS. 1 are photographs showing results obtained by examining efficiency of introduction of GFP into rice radicles using various cell-penetrating peptides (Example 1).
FIGS. 2 are graphs showing results obtained by examining efficiency of introduction of GFP into rice radicles, rice protoplasts, tobacco leaves, and *Arabidopsis* leaves using various cell-penetrating peptides (Example 1).
FIGS. 3 are graphs each showing results obtained by examining effects of a molar ratio of GFP and a cell-penetrating peptide in a mixed solution on the efficiency of introduction of GFP (Example 1) .
FIGS. 4 are photographs showing results obtained by examining effects of the kind and pH of buffers in mixed solutions on efficiency of introduction of GFP (Example 1).
FIG. 5 is a photograph showing results of separation of mHd3a-1 capable of high level expression by SDS-PAGE (Example 2) .
FIG. 6 is a graph showing results of an analysis of florigen functions of various mutated Hd3a's capable of high level expression (Example 2).
FIGS. 7 are photographs showing results obtained by examining efficiency of introduction of GFP in introduction of GFP into a rice shoot apical tissue using a cell-penetrating peptide (Comparative Example 1).
FIGS. 8 are photographs showing localization of a florigen in introduction of the florigen into a rice shoot apical tissue using a cell-penetrating peptide (Example 3).
FIGS. 9 are photographs showing results of introduction of a florigen into shoot apical tissues of corn and *Arabidopsis* using a cell-penetrating peptide (Example 4).
FIG. 10 is a graph showing results obtained by examining expression of a flower-bud formation marker gene in introduction of a florigen into a shoot apical tissue of wild-type rice (Example 5) .
FIG. 11 is a graph showing results obtained by examining expression of a flower-bud formation marker gene in introduction of a florigen into a shoot apical tissue of rice in which florigens have been inactivated (Example 5).
FIGS. 12 are photographs of an *Arabidopsis* flower bud induced by direct introduction of a florigen (Example 6).
FIG. 13 is a graph showing effects of promoting formation of a flower bud in direct introduction of a florigen into *Arabidopsis* (Example 6).
FIGS. 14 are graphs showing effects on induction of expression of a flowering-responsive gene in direct introduction of a florigen into *Arabidopsis* (Example 7).

### Description of Embodiments

The present invention is directed to a method for promoting flowering of a plant comprising using a method of introducing a florigen into a cell of a shoot apical tissue of a plant using a cell-penetrating peptide, comprising the step of bringing a mixed solution obtained by mixing a florigen and a cell-penetrating peptide in an aqueous medium into contact with the shoot apical tissue of the plant.

In the present invention, the plant refers to a higher plant in which the florigen can act as a factor for promoting flowering. In general, the plant has a vegetative growth stage and a reproductive growth stage, and forms a flower bud upon transition of the growth phase from the vegetative growth to the reproductive growth. The phenomenon of transition from the vegetative growth to the reproductive growth is referred to as "flowering". For example, the flowering in a monocotyledon such as rice or wheat is ear emergence, and the ear emergence is a phenomenon in which an ear appears from a leaf sheath of a flag leaf by rapidly extending an internode (neck of spike) under a spike. The ear emergence is defined as a time when an ear tip appears in rice or as a time when the base of a spike completely appears in wheat. In addition, the flowering in a dicotyledon, *Arabidopsis,* is bolting, and a phenomenon in which extraction of rosette leaves is stopped to initiate extension of the scape is referred to as flowering.

The plant in the present invention may be a monocotyledon or a dicotyledon, or may be a short-day plant that forms a flower bud when the hours of daylight per day is shorter than a predetermined time period, a long-day plant that forms a flower bud when the hours of daylight per day is longer than a predetermined time period, or a day-neutral plant, the flowering of which is not affected by the hours of daylight per day. Specific examples of the plant in the present invention include a plant belonging to the family Brassicaceae, a plant belonging to the family Gramineae, a plant belonging to the family Solanaceae, a plant belonging to the family Leguminosae, a plant belonging to the family Rosaceae, a plant belonging to the family Rutaceae, and a plant belonging to the family Anacardiaceae. Examples of the plant belonging to the family Brassicaceae include *Arabidopsism,* cabbage, and napa cabbage. Examples of the plant belonging to the family Gramineae include corn, rice, wheat, and barley. Examples of the plant belonging to the family Solanaceae include tobacco and tomato. An example of the plant belonging to the family Leguminosae is soybean. Examples of the plant belonging to the family Rosaceae include various rose garden varieties, strawberry, apple, and almond. Examples of the plant belonging to the family Rutaceae include orange, kumquat, and hardy orange. An example of the plant belonging to the family Anacardiaceae is mango. The plant in the present invention is preferably a plant belonging to the family Gramineae or a plant belonging to the family Brassicaceae, more preferably rice or *Arabidopsis.*

The flowering occurs in a shoot apical tissue (meristem) of a plant. The shoot apical tissue is an apical meristem of a plant body and is present at the peripheral part of a stem. In the shoot apical tissue, new cells are born at all times by cell division to form tissues such as stems, leaves, and flower buds. When the plant undergoes transition from the vegetative growth to the reproductive growth, the shoot apical tissue is converted into an inflorescence meristem to initiate formation of a flower bud. The florigen is considered to be transported from the leaf to the shoot apical tissue and to induce the flowering in the shoot apical tissue.

In the present invention, the florigen is not particularly limited, and examples thereof include rice Hd3a (Os06g0157700) (Genbank Accession No. BAB61028: derived from Oryza *sativa Japonica* group cultivar Nipponbare), rice RFT1 (Genbank Accession No. AB062676), *Arabidopsis* FT (Genbank Accession No. AB027504), *Arabidopsis* TSF (Genbank Accession No. AB027506), tomato SFT (Genbank Accession No. AY186735), and wheat VRN3 (Genbank Accession No. LOC100037541). Of those, rice Hd3a is preferred. In addition, the florigen in the present invention can be obtained by expressing a high level of the florigen by a genetic recombination technology and purifying the florigen, and is preferably one that is highly soluble and has a function of a flowering promoting factor. An example of the florigen is a mutated florigen obtained by introducing a mutation of a substitution of from 1 to 10 (preferably from 1 to 7, more preferably from 1 to 3) amino acids in an amino acid moiety exposed on the outer surface of the florigen, or a partial peptide of the mutated florigen. Examples of the amino acid moiety exposed on the outer surface of the florigen include amino acids corresponding to lysine at position 31, cysteine at position 43, glutamic acid at position 57, cysteine at position 109, tyrosine at position 136, tryptophan at position 140, and cysteine at position 166 in the amino acid sequence set forth in SEQ ID NO: 1. In the present invention, the "site of a protein corresponding to a specific amino acid (such as lysine at position 31) in SEQ ID NO: X" is meant to encompass, in addition to the site of a specific amino acid (such as lysine at position 31) in SEQ ID NO: X, a site corresponding to the specific amino acid (such as lysine at position 31) in a protein having an amino acid sequence other than SEQ ID NO: X and having a function equivalent to that of the protein of SEQ ID NO: X.

The mutated florigen obtained by introducing an amino acid substitution to a florigen or a partial peptide thereof is preferably mutated Hd3a or a partial peptide thereof, more preferably any of the following mutated florigens (1) to (5):
(1) a mutated florigen having an amino acid sequence set forth in SEQ ID NO: 1 with substitutions of amino acids corresponding to lysine at position 31 and glutamic acid at position 57;
(2) a mutated florigen having an amino acid sequence set forth in SEQ ID NO: 1 with substitutions of amino acids corresponding to cysteine at position 43, cysteine at position 109, and cysteine at position 166;
(3) a partial peptide of the mutated florigen described in (2), which includes an amino acid sequence corresponding to a sequence of amino acids at from position 6 to position 170 in the amino acid sequence set forth in SEQ ID NO: 1;
(4) a mutated florigen having an amino acid sequence set forth in SEQ ID NO: 1 with substitutions of amino acids corresponding to lysine at position 31, tyrosine at position 136, and tryptophan at position 140; and
(5) a partial peptide of the mutated florigen described in (4), which includes an amino acid sequence corresponding to a sequence of amino acids at from position 8 to position 170 in the amino acid sequence set forth in SEQ ID NO: 1.

In the present invention, as the mutated Hd3a or the partial peptide thereof, the one described in (1), (3), or (5) is preferably used, and the one described in (1) is more preferably used.

Specific examples of the mutated florigens (1) to (5) include various mutated Hd3a's described in Examples below, such as mHd3a-1 (full-length, having K31A and E57A amino acid substitutions), mHd3a-2T (a partial peptide having a sequence consisting of amino acids at positions from 6 to 170, having C43L, C109S, and C166S amino acid substitutions), or a partial peptide of the mutated Hd3a, i.e., mHd3a-3T (a partial peptide having a sequence consisting of amino acids at positions from 8 to 170, having K31A, Y136A, and W140A amino acid substitutions), mHd3a-2F (full-length, having C43L, C109S, and C166S amino acid substitutions), and m-Hd3a-3F (full-length, having K31A, Y136A, and W140A amino acid substitutions).

The cell-penetrating peptide refers to a peptide having cell membrane permeability, and is classified based on chemical properties into three groups including a peptide characterized by containing many basic amino acids (in particular, arginine and lysine), an amphiphilic peptide, and a peptide having an RXLR sequence. Examples of the cell-penetrating peptide may include Tat (SEQ ID NO: 2), FHV coat (SEQ ID NO: 3), Pep-1 (SEQ ID NO: 4), Penetratin (SEQ ID NO: 5), Transportan (SEQ ID NO: 6), R9 (SEQ ID NO: 7), KALA (SEQ ID NO: 8), Avr1b (SEQ ID NO: 9), gp41 (SEQ ID NO: 11), Rev (SEQ ID NO: 22), pAntp (SEQ ID NO: 10), VP22 (SEQ ID NO: 13), SV40NLS (SEQ ID NO: 12), Integrin β3 (SEQ ID NO: 14), Influenza HA-2 (SEQ ID NO: 15), R/W (SEQ ID NO: 18), BMV-gag (SEQ ID NO: 16), HTLV-II Rex (SEQ ID NO: 17), Human cFOS (SEQ ID NO: 19), Human cJUN (SEQ ID NO: 20), and Yeat GCN4 (SEQ ID NO: 21) (see Table 1 and Table 2 below). In the present invention, the cell-penetrating peptide is preferably selected from an arginine-rich peptide (preferably, a peptide including from 8 to 12 consecutive arginine residues) and a peptide having an RXLR sequence. The peptide including from 8 to 12 consecutive arginine residues is most preferably a peptide including 9 consecutive arginine residues (R9). The peptide having an RXLR sequence is most preferably Avr1b.

**[Table 1]**

| Name | Sequence | Chemical properties | Characteristics |
|---|---|---|---|
| Tat | YGRKKRRQRRR (SEQ ID NO: 2) | Arginine-ric h | Derived from Tat protein of HIV virus |
| FHV coat | RRRRNRTRRNRRRVR (SEQ ID NO: 3) | Arginine-ric h | Derived from coat protein of FHV virus |
| Pep-1 | KETWWETWWTEWSQPKKKRKV (SEQ ID NO: 4) | Amphiphilic | Artificially designed sequence |
| Penetratin | RQIKIWFQNRRMKWKK (SEQ ID NO: 5) | Arginine and lysine-rich | Derived from Drosophila Antennapedia protein |
| Transportan | GWTLNSAGYLLGKINLKALAALAKKIL (SEQ ID NO: 6) | Amphiphilic | Derived from neuronal protein galanin |
| R9 | RRRRRRRRR (SEQ ID NO: 7) | Arginine-ric h | Artificially designed sequence |
| KALA | | Amphiphilic | Artificially designed sequence |
| Avr1b | | Having RXLR sequence | Derived from potato phytophthora effector protein |

**[Table 2]**

| Name | Sequence | Characteristics | Derivation |
|---|---|---|---|
| p-Antp | RQIKIWFQNRRMKWKK (SEQ ID NO: 10) | Amphiphilic | Derived from Drosophila transcription factor |
| gp41 | GALFLGFLGAAGSTMGA (SEQ ID NO: 11) | Amphiphilic | Derived from glycoprotein 41 |
| SV40NLS | PKKKRKV (SEQ ID NO: 12) | Arginine-ric h | Derived from Simian Virus 40 nuclear transport signal sequence |
| VP22 | | Arginine-ric h | Derived from herpes simplex virus VP22 protein |
| Integrin β3 | VTVLALGALAGVGVG (SEQ ID NO: 14) | Amphiphilic | Derived from Integrin β3 protein |
| Influenza HA-2 | | Lysine-rich | Derived from influenza virus HA protein |
| BMV-gag | KMTRAQRRAAARRNRWTA (SEQ ID NO: 16) | Arginine-ric h | Derived from brome mosaic virus (BMV) Gag protein |
| HTLV-II Rex | TRRQRTRRARRNR (SEQ ID NO: 17) | Arginine-ric h | Derived from human T-cell lymphotrophic virus (HTLV)-II Rex protein |
| R/W | RRWWRRWRR (SEQ ID NO: 18) | Arginine-ric h | Artificially designed peptide |
| Human c Fos | KRRIRRERNKMAAAKSRNRRRELTDTGC (SEQ ID NO: 19) | Arginine-ric h | Derived from human cFOS protein |
| Human c Jun | | Arginine-ric h | Derived from human cJun protein |
| Yeast GCN4 | KRARNTEAARRSRARKLQRMKQGC (SEQ ID NO: 21) | Arginine-ric h | Derived from yeast transcription factor |
| Rev | TRQARRNRRRRWRERQR (SEQ ID NO: 22) | Arginine-ric h | HIV rev protein |

The method of the present invention may include the step of bringing the florigen into contact with the shoot apical tissue of the plant in a state where the florigen and the cell-penetrating peptide coexist. The state where the florigen and the cell-penetrating peptide coexist may be a state where the florigen and the cell-penetrating peptide are present in the same solvent. The florigen and the cell-penetrating peptide may be present in a solvent as independent molecules, or the florigen and the cell-penetrating peptide may be present in a solvent as one molecule including the florigen and the cell-penetrating peptide, such as a fusion protein. The introduction method of the present invention preferably includes the step of bringing the florigen and cell-penetrating peptide in a state where the florigen and the cell-penetrating peptide are present in one solvent as independent molecules, that is, a solution obtained by mixing the florigen and the cell-penetrating peptide in an aqueous medium into contact with the shoot apical tissue of the plant. It should be noted that, in this description, the solution containing the florigen and the cell-penetrating peptide in one solvent is referred to as "solution for florigen introduction".

An example of the molecule including the florigen and the cell-penetrating peptide is a fusion protein. The fusion protein can be prepared by a hitherto known method, and can be obtained by introducing a gene obtained by fusing a gene coding for the florigen and a gene coding for the cell-penetrating peptide into a cell of a host such as yeast or *Escherichia coli,* expressing the fusion protein in the cell, and isolating and purifying the fusion protein. The amino acid substitution in the florigen can be introduced by a site-directed mutagenesis method well known to a person skilled in the art, and may be introduced using a commercially available kit or the like (such as QuikChangeTM Site-Directed Mutagenesis Kit (STRATAGENE)).

In the case where the florigen and the cell-penetrating peptide are prepared as independent molecules, the cell-penetrating peptide to be used can be obtained by chemical synthesis through the use of a hitherto known technology or may be a commercial available product.

The florigen is preferably obtained by, through the use of a hitherto known technology, introducing a gene coding for the florigen into a cell of a host such as yeast or *Escherichia coli,* expressing the florigen in the cell, and isolating and purifying the florigen. A peptide fragment may be added to the florigen so as not to affect the function of the florigen depending on the expression system of the host cell. For example, in the case of using the expression system of *Escherichia coli,* a peptide fragment (GPGHM) derived from an *Escherichia coli* expression plasmid is added to the N-terminal of the protein. The amino acid substitution in the florigen can be introduced by a site-directed mutagenesis method well known to a person skilled in the art, and may be introduced using a commercially available kit or the like (such as QuikChangeTM Site-Directed Mutagenesis Kit (STRATAGENE)) .

The solution for florigen introduction including the florigen and the cell-penetrating peptide as independent molecules can be prepared by mixing the resultant florigen and cell-penetrating peptide in a solvent. The solution thus prepared is referred to as "mixed solution of the florigen and the cell-penetrating peptide" (sometimes simply referred to as "mixed solution").

The amounts of the florigen and the cell-penetrating peptide in the solution for florigen introduction are as follows from the viewpoint of efficiency of introduction of the florigen. When the molar amount of the florigen is defined as 1, the molar amount of the cell-penetrating peptide is required to be 1 or more. The molar ratio of the florigen and the cell-penetrating peptide in the mixed solution is preferably from 1:1 to 1:50, more preferably from 1:1 to 1:10.

The final concentration of the florigen in the solution for florigen introduction is preferably from 5 to 500 µM, more preferably from 10 to 100 µM. The final concentration of the cell-penetrating peptide in the mixed solution is preferably from 5 to 5,000 µM, more preferably from 10 to 1,000 µM. When the concentrations of the florigen and the cell-penetrating peptide in the solution for florigen introduction are too high, an osmotic stress may be generated to damage cells of the plant, which is not preferred.

The solvent in the solution for florigen introduction is preferably an aqueous medium. The aqueous medium refers to water or an aqueous solution such as a buffer. The aqueous medium to be used in the present invention is preferably a buffer. The buffer encompasses acetate buffer, phosphate buffer, citrate buffer, borate buffer, tartrate buffer, Tris buffer, phosphate buffered saline (PBS), and the like, and further encompasses a medium to be generally used for cell culture, such as Murashige and Skoog medium (MS medium). Phosphate buffered saline (PBS) or a solution obtained by diluting the PBS to any concentration up to one-tenth of the concentration of the PBS is preferably used as the aqueous medium of the present invention from the viewpoint of efficiency of introduction of the florigen. More preferably, there is used PBS having a composition of 137 mM NaCl, 2.7 mM KCl, 10 mM Na₂HPO₄, and 1.76 mM KH₂PO₄ or a solution obtained by diluting the PBS to any concentration up to one-tenth of the concentration of the PBS. The degree of the dilution only needs to be suited for growth of the plant, and the PBS having the composition is preferably diluted to a concentration of from one-fifth to one-tenth of the concentration of the PBS. In addition, the aqueous medium has a pH of preferably from 5.8 to 9.0, more preferably from 6.5 to 8.0, from the viewpoint of efficiency of introduction of the florigen.

Any substance may be added to the solution for florigen introduction as long as the substance does not reduce the efficiency of introduction of the florigen and the cell-penetrating peptide into the tissue.

The method of the present invention may include the step of exposing the shoot apical tissue in order to bring the solution for florigen introduction into contact with the shoot apical tissue. When, for example, the solution for florigen introduction is added dropwise after the shoot apical tissue is exposed, the solution for florigen introduction can be brought directly into contact with the shoot apical tissue. Alternatively, the shoot apical tissue may be brought into contact with the solution for florigen introduction without exposing the shoot apical tissue by sprinkling an enough amount of the solution for florigen introduction to the vicinity of the shoot apical tissue or by injecting the solution for florigen introduction into the vicinity of the shoot apical tissue using an injector or the like. Alternatively, the solution for florigen introduction may be transported to the shoot apical tissue through the sieve tube of the plant by, for example: absorbing the solution for florigen introduction via an incision of the stem, petiole, leaf, joint, root, or the like of the plant; introducing the solution for florigen introduction based on a capillary phenomenon via a thread passed to the vicinity of the stem or shoot apical tissue; or immersing the whole of the plant in the solution for florigen introduction.

The present invention encompasses a promotion method for flowering of a plant according to appended claim 1. In the cell of the shoot apical tissue, the florigen is considered to be bound to 14-3-3 protein and bZIP transcription factor to form a florigen activation complex and to remain in the nucleus. When the process is started, the florigen activation complex is accumulated in the nucleus. After that, the florigen activation complex activates transcription of a flower-bud formation marker gene (for example, OsMADS15 gene) that causes induction of flowering, resulting in promoting flowering of a plant. In the promotion method for flowering of a plant of the present invention, introduction of the florigen into the shoot apical tissue causes activation of transcription of the flower-bud formation marker gene (for example, OsMADS15 gene), resulting in promoting flowering. In addition, in formation of a flower bud, expression of flowering-responsive genes such as APETALA1 (AP1) gene, FRUITFULL (FUL) gene, and SQUAMOSA PROMOTER BINDING-LIKE3 (SPL3) gene is induced. The expression of FUL gene is induced in the shoot apical tissue upon transition from the vegetative stage to the reproductive stage, and the expression of AP1 gene is induced in primordia of floral bud immediately after its differentiation from the shoot apical tissue in the reproductive stage. The SPL3 gene is a gene that is located genetically upstream of the AP1 gene and the FUL gene and is induced in the early stage of the flowering. In the promotion method for flowering of a plant of the present invention, the florigen is introduced into the shoot apical tissue to induce expression of the SPL3 gene, AP1 gene, and FUL gene. According to the promotion method for flowering of a plant of the present invention, transcription of the flowering marker gene and the flowering-responsive gene is activated to form a flower bud primordium in the shoot apical tissue, as compared to the case where the florigen is not introduced, resulting in promoting flowering.

The present invention further encompasses a use of an agent for promoting flowering of a plant by introducing a florigen into a cell of a shoot apical tissue of a plant, the agent comprising:
a florigen; and
a cell-penetrating peptide,
wherein the agent is for contacting with the shoot apical tissue of the plant. The agent for introducing a florigen includes preferably a solution for florigen introduction, in which the florigen and the cell-penetrating peptide are present in the same aqueous medium, more preferably a mixed solution obtained by mixing the florigen and the cell-penetrating peptide. In addition, the present invention further encompasses an agent for promoting flowering of a plant, which includes the agent for introducing a florigen.

The present disclosure further encompasses a kit for introducing a florigen into a cell of a shoot apical tissue of a plant, which includes a florigen and a cell-penetrating peptide. The kit may include the florigen and the cell-penetrating peptide as independent molecules or as one molecule such as a fusion protein. The kit may include an aqueous medium for mixing the florigen and the cell-penetrating peptide, or may further include another additive. In the case where the kit includes the florigen and the cell-penetrating peptide as independent molecules, the florigen and the cell-penetrating peptide may be mixed to prepare a mixed solution immediately before introduction of the florigen into a plant.

### Examples

The present invention is hereinafter described by way of Examples and Comparative Examples. However, it should be appreciated that the present invention is not limited to the description of Examples.

### (Example 1) Study on method of introducing protein into plant tissue cell

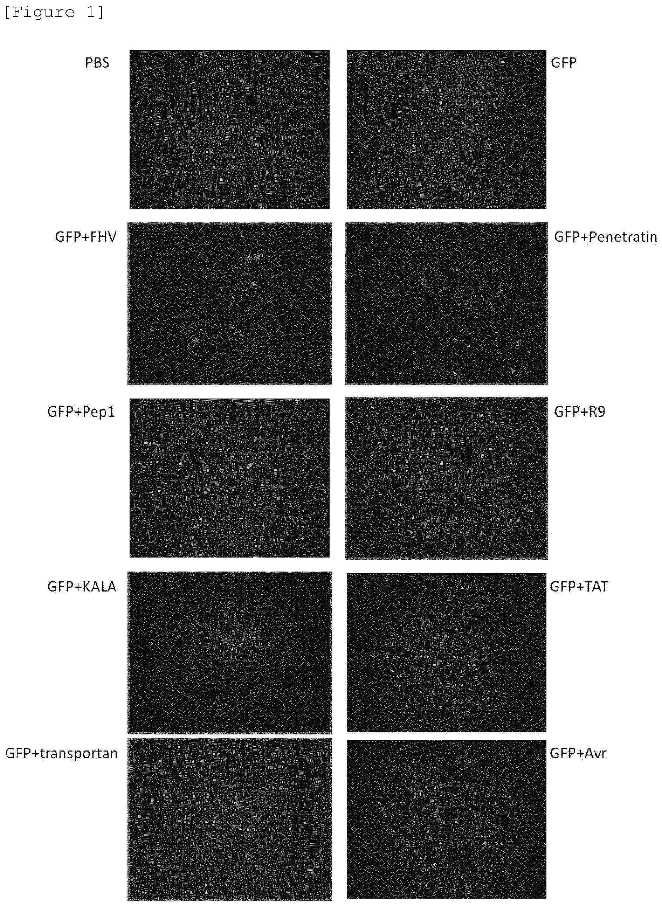

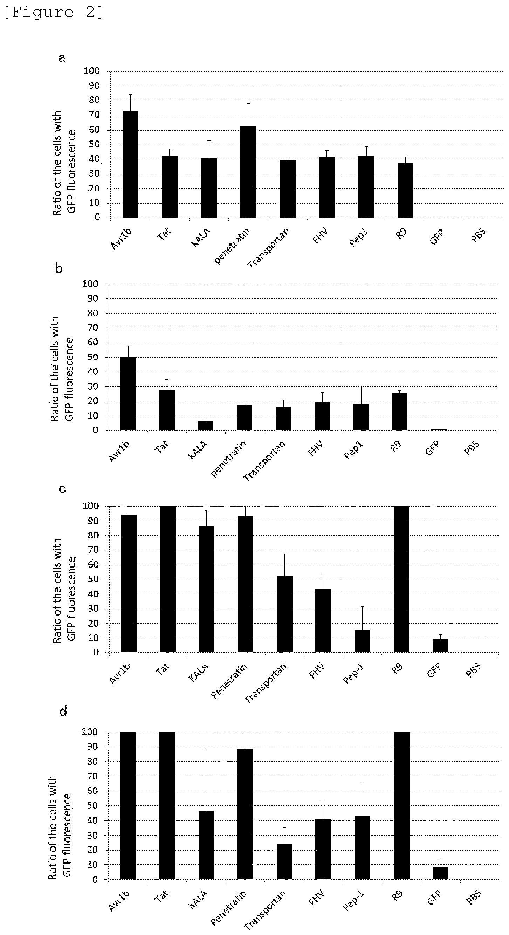

(1) A cell-penetrating peptide and a protein were separately prepared and mixed, and a plant tissue was treated with the mixture.
   Eight kinds of cell-penetrating peptides shown in Table 1 above and GFP were separately mixed in PBS (137 mM NaCl, 2.7 mM KCl, 10 mM Na₂HPO₄, 1.76 mM KH₂PO₄) (pH 7.4) so as to achieve a final concentration of 50 µM, and rice radicles were immersed in the mixtures. The eight kinds of cell-penetrating peptides are typical peptides selected from arginine-rich peptides, amphiphilic peptides, and peptides each having an RXLR sequence, and were prepared by chemical synthesis. The immersion treatment was carried out for 16 hours at room temperature, followed by low-temperature treatment at 4°C for 4 hours. The samples were washed until fluorescence was not observed in a negative control group treated with GFP alone, and cells were counted under a fluorescence stereomicroscope to calculate ratios of fluorescent cells. As a result, it was found that this method was able to efficiently introduce GFP directly into a cell of a plant (FIGS. 1).
(2) The efficiency of introduction of GFP by the eight kinds of cell-penetrating peptides was examined quantitatively in the same manner as in (1) above. Rice radicles (FIG. 2a), cultured rice cells (rice protoplasts) (FIG. 2b), tobacco leaves (FIG. 2c), and *Arabidopsis* leaves (FIG. 2d) were used as tissues to be examined. A total of 32 treatment groups were evaluated. As a result, comprehensive efficiency of introduction by Avr1b was the highest, and the efficiency of introduction by R9 and Tat were the second highest.
(3) The effect of the molar ratios of the cell-penetrating peptides to the protein on the efficiency of introduction of the protein was examined. The molar ratios of the cell-penetrating peptides (Avrlb and R9) to the protein were changed to 0.01, 0.1, 1, and 10, and the efficiency of introduction of GFP into rice root epidermal cells was evaluated.

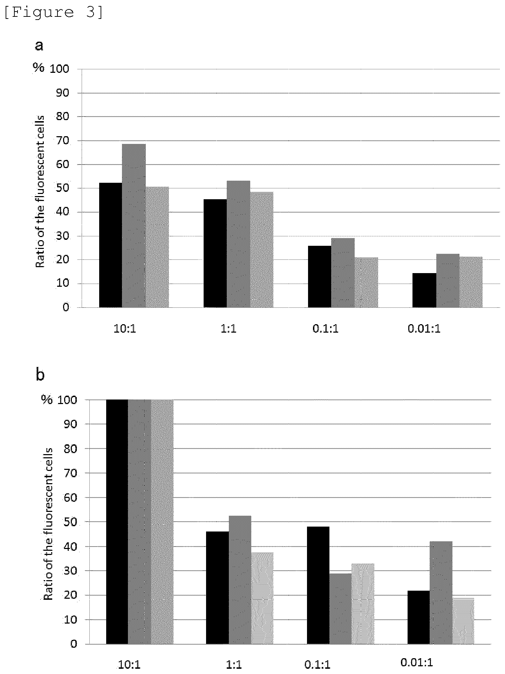

   FIGS. 3 show the results. FIG. 3a shows results of the test using the mixed solution of Avr1b and GFP, and FIG. 3b shows results of the test using the mixed solution of R9 and GFP. As the molar ratio of R9 increased in the order of 0.01, 0.1, 1, and 10, the introduction efficiency increased. When R9 was used at a molar ratio 10 times higher than that of the florigen, the efficiency of introduction of GFP increased 2.5 times as compared to the case of using R9 at a molar ratio of 1 times. As the molar ratio of Avr1b increased in the order of 0.01, 0.1, 1, and 10, the introduction efficiency increased although the increase rate was small as compared to R9.
(4) There was examined the effect of conditions such as the kind and pH of a medium in which the protein and the cell-penetrating peptide were mixed on the efficiency of introduction of the protein. MS media (three kinds: pH 5.8, 6.5, and 7.4) and PBS's (five kinds: pH 5.8, 6.5, 7.4, 8.0, and 9.0) were used as media to be used for treatment with the cell-penetrating peptide and the protein to examine the efficiency of direct introduction of GFP into rice radicles. The MS media were prepared using "Plant tissue medium series, Mixed salts for Murashige and Skoog medium" (manufactured by NIHON PHARMACEUTICAL CO., LTD.). The pH's of the MS media were adjusted using hydrochloric acid or an aqueous sodium hydroxide solution. In addition, as the PBS's, PBS's having the same compositions as that in Example 1 were used, and the pH's were adjusted using hydrochloric acid or an aqueous sodium hydroxide solution.

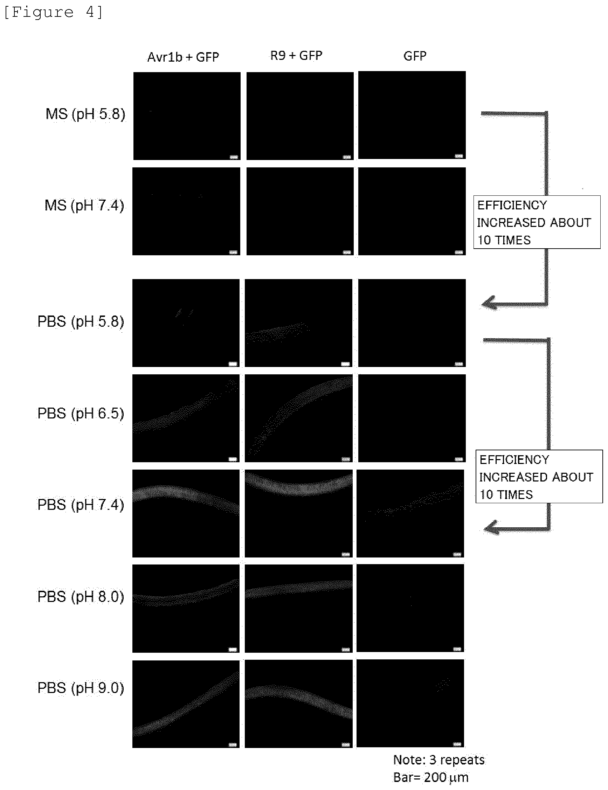

FIGS. 4 show the results. In the case of using the PBS (pH 5.8), the efficiency of introduction of GFP increased 10 times as compared to the case of using the MS medium (pH 5.8). Further, in the case of using the PBS (pH 7.4), the efficiency of introduction of GFP increased 10 times as compared to the case of using the PBS (pH 5.8) . Comprehensively, the highest efficiency of introduction was achieved in the case of using the PBS (pH 7.4).

### (Example 2) Search for florigen capable of high level expression

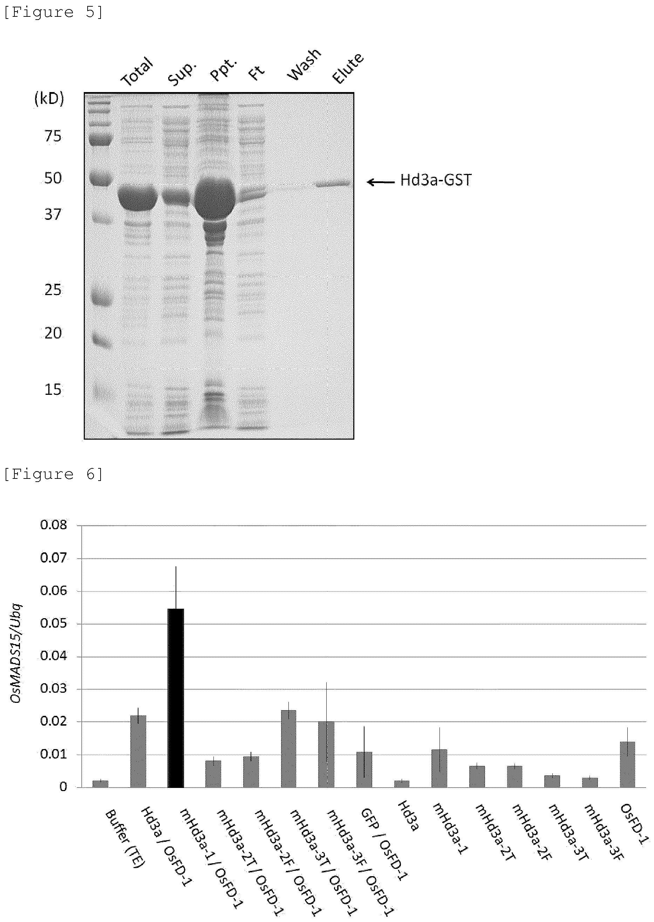

(1) Wild-type Hd3a (SEQ ID NO: 1) cannot be synthesized in a large amount under usual conditions, and hence a mutation to stabilize Hd3a and to increase the yield was introduced into Hd3a, followed by expression (see Non Patent Literature 2).
   The results revealed that three kinds of florigens, i.e., mHd3a-1 (full-length, having K31A and E57A amino acid substitutions), mHd3a-2T (a partial peptide having a sequence consisting of amino acids at positions from 6 to 170, having C43L, C109S, and C166S amino acid substitutions), and mHd3a-3T (a partial peptide having a sequence consisting of amino acids at positions from 8 to 170, having K31A, Y136A, and W140A amino acid substitutions), were capable of high level expression and providing soluble fractions. FIG. 5 is a photograph showing mHd3a-1 separated and detected by SDS-PAGE through a conventional method.
(2) The florigen functions were examined for mutated Hd3a partial peptides of mHd3a-2F (full-length, having C43L, C109S, and C166S amino acid substitutions) and m-Hd3a-3F (full-length, having K31A, Y136A, and W140A amino acid substitutions) in addition to the mutated Hd3a's examined in (1) above, i.e., mHd3a-1, mHd3a-2T, and mHd3a-3T. The assay system used is a transient expression system for protoplasts. When the mutated Hd3a and a transcription factor OsFD1 are transiently expressed in protoplasts in this system, a flower-bud formation marker gene OsMADS15 is transcriptionally activated if the Hd3a is functional. The transient expression system for protoplasts was constructed according to the method described in Non Patent Literature 2. In addition, the expression of the OsMADS15 gene was examined by RT-PCR method according to the method described in Non Patent Literature 2.

The test was carried out using the mutated Hd3a's and partial peptides thereof, and the results revealed that mHd3a-1 most desirably activated transcription of the OsMADS15 gene (FIG. 6).

### (Comparative Example 1) Introduction of GFP into cell of rice shoot apical tissue

A rice shoot apical tissue was exposed under a stereomicroscope, and a cell-penetrating peptide and GFP were separately prepared and mixed in the same manner as in Example 1 and were used for treatment of the plant tissue. A mixed solution was prepared by adding GFP at a final concentration of 50 µM and a cell-penetrating peptide R9 at a final concentration of 500 µM in PBS (pH 7.4). The mixed solution was added dropwise to the exposed shoot apical tissue to bring the mixed solution into contact with the tissue. The tissue was treated for 16 hours at room temperature.

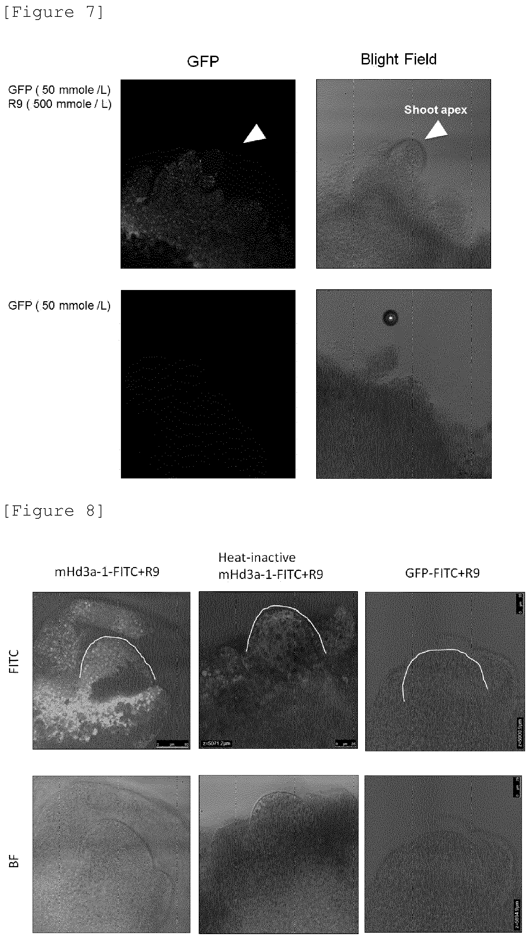

As a result, it was found that the efficiency of introduction of GFP into the shoot apex meristem was not so high (FIGS. 7).

### (Example 3) Introduction of florigen into cell of rice shoot apical tissue

The mHd3a-1 prepared by high level expression in Example 2 was labeled with a fluorochrome FITC. Subsequently, a rice shoot apical tissue was exposed under a stereomicroscope, and the cell-penetrating peptide and the florigen were mixed and used for treatment of the plant tissue in the same manner as in Example 1. A mixed solution was prepared by adding the florigen at a final concentration of 50 µM and the cell-penetrating peptide R9 at a final concentration of 500 µM in PBS (pH 7.4). The mixed solution was added dropwise to the exposed shoot apical tissue to bring the mixed solution into contact with the tissue. The tissue was treated for 16 hours at room temperature.

The results revealed that the mHd3a-1 was introduced efficiently into the rice shoot apical tissue (FIGS. 8). The results further revealed that the mHd3a-1 was localized accurately in the nucleus serving as a functional site of the florigen in the cell of the shoot apical tissue.

### (Example 4) Introduction of florigen into cells of shoot apical tissues of corn and Arabidopsis

In the same manner as in Example 3, the mixed solution of the cell-penetrating peptide and the florigen was added dropwise to shoot apical tissues of corn and *Arabidopsis* to treat the tissues.

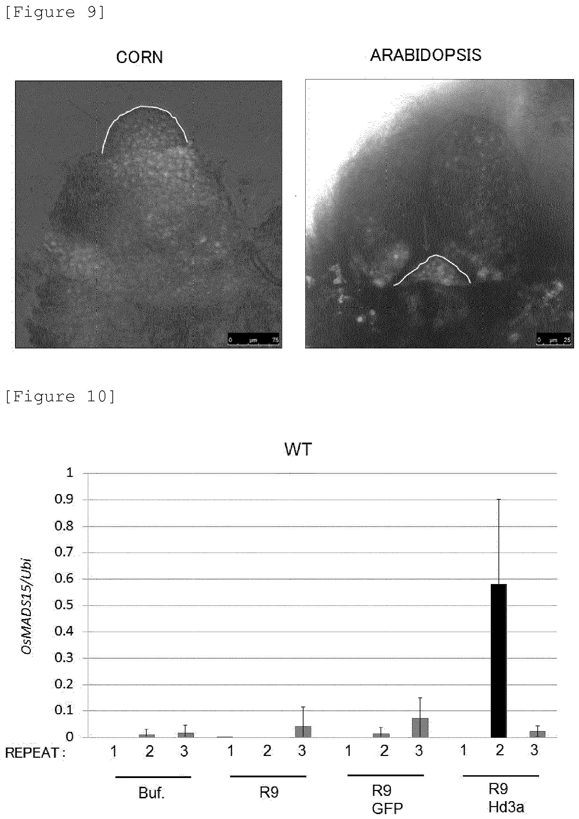

The results revealed that the mHd3a-1 was introduced efficiently into the shoot apical tissues of corn and *Arabidopsis* (FIGS. 9). The results further revealed that the mHd3a-1 was localized accurately in the nucleus serving as a functional site of the florigen in the cell of the shoot apical tissue.

### (Example 5) Examination of effect of direct introduction of florigen on expression of flower-bud formation marker gene

(1) A rice shoot apical tissue into which the florigen was introduced was isolated in the same manner as in Example 3. RNA was extracted from the rice shoot apical tissue by a conventional method, and expression of a flower-bud formation marker gene (OsMADS15 gene) was examined by real-time quantitative RT-PCR (see Non Patent Literature 2 for the method).
   The results revealed that direct introduction of the florigen into the shoot apical tissue can activate expression of the flower-bud formation marker gene (OsMADS15 gene) (FIG. 10).
(2) Further, rice in which all florigens were inactivated (Hd3a-RFT1 double RNAi) (Komiya et al. (2008) Development 135:767-774) was subjected to the same experiment as in (1). Rice has two florigen genes (Hd3a and RFT1), and flower bud-forming ability is completely lost in the rice in which expression of both the genes has been simultaneously suppressed (Hd3a-RFT1 double RNAi). Therefore, the rice usually exhibits almost no expression of the flower-bud formation marker gene.

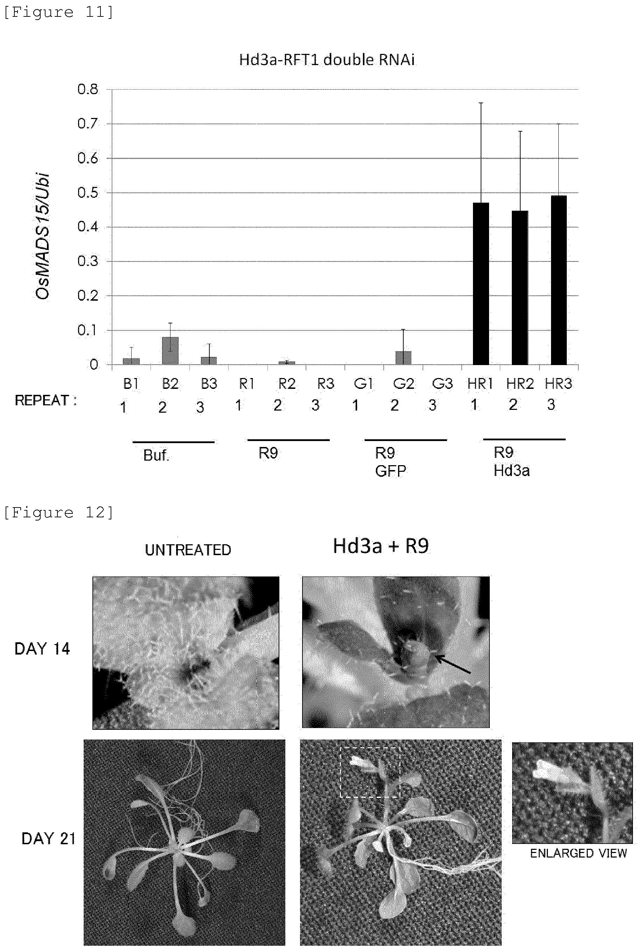

It was found that direct introduction of the florigen even into the rice in which all florigens were inactivated (Hd3a-RFT1 double RNAi) could activate the expression of the flower-bud formation marker gene (FIG. 11).

### (Example 6) Examination of flowering-promoting effect in plant body into which florigen has been directly introduced

A mixed solution prepared by mixing the florigen mHd3a-1 and the cell-penetrating peptide R9 in ten-times diluted PBS in the same manner as in Example 3 was used for treatment of seedling of *Arabidopsis* at the time of cotyledon expansion about three days after seeding. It should be noted that the shoot apices in seedling of *Arabidopsis* was exposed at the time of cotyledon expansion. The treatment was carried out at room temperature for 2 days. After that, the mixed solution was washed, and the plants were cultivated on the MS medium under a short-day condition (day length: 10 hours) for from 30 to 40 days to examine the timing of the flowering. The examination condition of the start of the flowering was defined as the start of bolting or a state where differentiation of flower buds was visually observed, and whether the flowering was early or late was evaluated based on the number of rosette leaves at the time when the state described above was achieved. In the case of *Arabidopsis,* rosette leaves are usually extracted, and the extraction of the rosette leaves is stopped when the flowering is started. When the flowering is started in the early stage, the number of the leaves is small because only a small number of the rosette leaves can be extracted, while when it takes a long time before the flowering is started, the number of the leaves is large because many rosette leaves can be extracted.

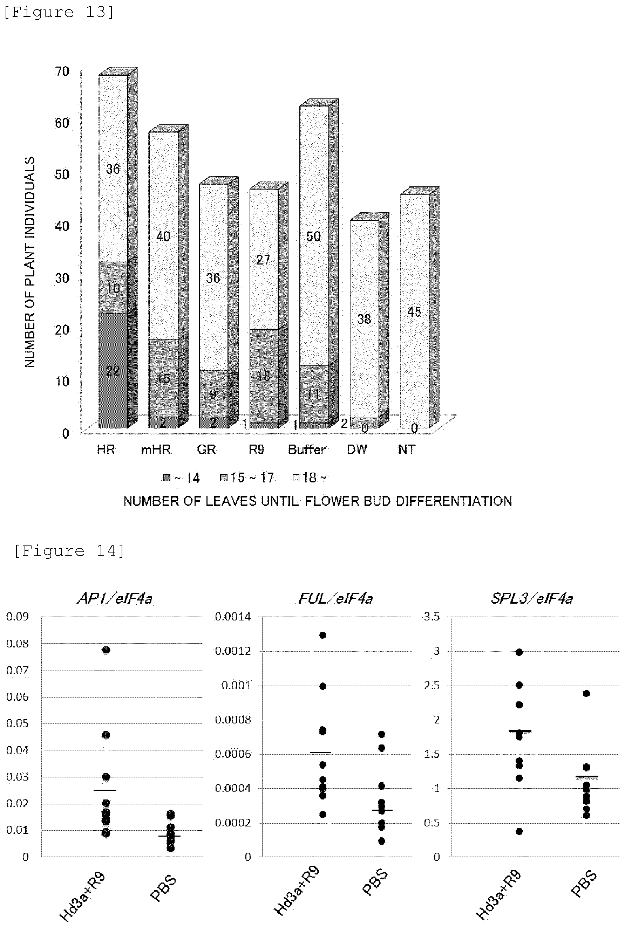

FIGS. 12 and FIG. 13 show the results. FIGS. 12 are photographs taken on day 14 and day 21, when the cultivation start date under the short-day condition is defined as day 0. The enlarged view is one obtained by enlarging the area enclosed by a dashed line in the photograph of a flower bud of an individual treated with the mixed solution of mHd3a-1 and R9, which was taken on day 21. In addition, the numbers in the graph of FIG. 13 represent numbers of individuals that achieved the flowering at each number of leaves. In FIG. 13, "HR" represents results of treatment with the mixed solution of mHd3a-1 and R9, "mHR" represents results of treatment with the mixed solution of mutated Hd3a incapable of being bound to a receptor (specifically, Hd3a R64D F103A R132D) and R9, "GR" represents results of treatment with the mixed solution of GFP and R9, "R9" represents results of treatment with a solution containing R9 alone, "Buffer" represents results of treatment with PBS alone, "DW" represents results of treatment with distilled water, and "NT" represents results of no treatment.

Almost all the untreated plants were not able to start the flowering under the conditions of this example. However, in the case of the treatment with mHd3a-1 and R9, about 30% of the plants were able to start the flowering when the number of the leaves was 14 or less. Even when the plants were treated with mutated Hd3a (mHR) incapable of being bound to a receptor or GFP instead of mHd3a-1, the effect of promoting the start of the flowering was not observed. Almost all the plants treated with R9 alone, PBS alone, and distilled water alone did not start the flowering when the number of the leaves was 14 or less. The results of the experiments revealed that direct introduction of the florigen into *Arabidopsis* could promote the flowering.

### (Example 7) Examination of effect on induction of expression of flowering-responsive gene in Arabidopsis into which florigen has been directly introduced

The florigen was introduced directly into *Arabidopsis* in the same manner as in Example 6, and then the plants were cultivated for 2 weeks, followed by collection of a shoot apical tissue from each individual. RNA was extracted from the shoot apical tissue collected from each individual, and cDNA was synthesized based on a reverse transcription reaction. After that, the expression levels of APETALA1 (AP1) gene, FRUITFULL (FUL) gene, and SQUAMOSA PROMOTER BINDING-LIKE3 (SPL3) gene were examined by real-time quantitative RT-PCR (see Yamaguchi et al. Dev. Cell 17:268-278 (2009)).

The results revealed that direct introduction of the florigen activated the expression of the three genes (FIGS. 14). Induction of the expression was observed in about 30% of the individuals treated, and the results corresponded to the ratio of the plants in which flowering was promoted in Example 6. It should be noted that eIF4a was used as an internal standard. In addition, the horizontal lines in FIGS. 14 represent average values.

### Industrial Applicability

According to the present invention, it is possible to alter the timing of flowering of a plant by using the solution for florigen introduction. The alteration of the timing of flowering of a plant is very useful in the fields of, for example, cultivation management and cultivar improvement of plants. The present invention can be carried out easily and can be applied to many plant species, and hence is expected to be put into practical use in the fields of plant breeding, cultivation of plants, biomass, and the like.

## Claims

1. A method for promoting flowering of a plant comprising using a method of introducing a florigen into a cell of a shoot apical tissue of a plant using a cell-penetrating peptide, comprising the step of bringing a mixed solution obtained by mixing a florigen and a cell-penetrating peptide in an aqueous medium into contact with the shoot apical tissue of the plant.

2. The method for promoting flowering of a plant according to claim 1, wherein the cell-penetrating peptide is selected from a peptide including from 8 to 12 consecutive arginine residues and a peptide having an RXLR sequence.

3. The method for promoting flowering of a plant according to claim 1 or 2, wherein the florigen comprises a mutated florigen having an amino acid sequence set forth in SEQ ID NO: 1 with a substitution of at least one amino acid selected from amino acids corresponding to lysine at position 31, cysteine at position 43, glutamic acid at position 57, cysteine at position 109, tyrosine at position 136, tryptophan at position 140, and cysteine at position 166, or a partial peptide thereof.

4. The method for promoting flowering of a plant according to any one of claims 1 to 3, wherein the florigen comprises a mutated Hd3a protein having an amino acid sequence set forth in SEQ ID NO: 1 with a substitution of from 1 to 10 amino acids, or a partial peptide thereof.

5. The method for promoting flowering of a plant according to any one of claims 1 to 4, wherein the florigen is selected from the following mutated florigens (1) to (5):
(1) a mutated Hd3a having an amino acid sequence set forth in SEQ ID NO: 1 with substitutions of lysine at position 31 and glutamic acid at position 57;
(2) a mutated Hd3a having an amino acid sequence set forth in SEQ ID NO: 1 with substitutions of cysteine at position 43, cysteine at position 109, and cysteine at position 166;
(3) a partial peptide of the mutated Hd3a described in (2), which includes a sequence of amino acids at from position 6 to position 170 in the amino acid sequence set forth in SEQ ID NO: 1;
(4) a mutated Hd3a having an amino acid sequence set forth in SEQ ID NO: 1 with substitutions of lysine at position 31, tyrosine at position 136, and tryptophan at position 140; and
(5) a partial peptide of the mutated Hd3a described in (4), which includes a sequence of amino acids at from position 8 to position 170 in the amino acid sequence set forth in SEQ ID NO: 1.

6. The method for promoting flowering of a plant according to any one of claims 1 to 5, wherein a molar ratio of the florigen and the cell-penetrating peptide in the mixed solution is from 1:1 to 1:50.

7. The method for promoting flowering of a plant according to any one of claims 1 to 6, wherein the aqueous medium has a pH of from 6.5 to 8.0.

8. The method for promoting flowering of a plant according to any one of claims 1 to 7, wherein the aqueous medium comprises phosphate buffered saline (PBS).

9. A use of an agent for promoting flowering of a plant by introducing a florigen into a cell of a shoot apical tissue of a plant, the agent comprising:
a florigen; and
a cell-penetrating peptide,
wherein the agent is contacted with the shoot apical tissue of the plant.

10. The use according to claim 9, wherein the agent comprises a mixed solution obtained by mixing the florigen and the cell-penetrating peptide in an aqueous medium.

11. Use of a kit for promoting flowering of a plant by introducing a florigen into a cell of a shoot apical tissue of a plant, the kit comprising:
a florigen; and
a cell-penetrating peptide,
wherein the agent is contacted with the shoot apical tissue of the plant in a state where the florigen and the cell-penetrating peptide coexist.

## Patentansprüche

1. Ein Verfahren zur Förderung des Blühens einer Pflanze, umfassend der Verwendung eines Verfahrens des Einführens eines Florigens in eine Zelle eines Sprossapikalgewebes einer Pflanze mittels eines Zell-penetrierenden Peptids, umfassend den Schritt des Inkontaktbringens einer gemischten Lösung, die durch Mischen eines Florigens und eines Zell-penetrierenden Peptids in einem wässrigen Medium erhalten wird, mit dem Sprossapikalgewebe der Pflanze.

2. Das Verfahren zur Förderung des Blühens einer Pflanze nach Anspruch 1, wobei das Zell-penetrierende Peptid ausgewählt wird aus einem Peptid, welches 8 bis 12 aufeinanderfolgende Argininreste enthält, und einem Peptid, welches eine RXLR-Sequenz aufweist.

3. Das Verfahren zur Förderung des Blühens einer Pflanze nach Anspruch 1 oder 2, wobei das Florigen ein mutiertes Florigen, welches eine Aminosäuresequenz, die in SEQ ID NO: 1 angegeben ist, mit einer Substitution von mindestens einer Aminosäure aufweist, welche aus den Aminosäuren entsprechend Lysin an Position 31, Cystein an Position 43, Glutaminsäure an Position 57, Cystein an Position 109, Tyrosin an Position 136, Tryptophan an Position 140 und Cystein an Position 166 ausgewählt werden, oder ein Teilpeptid davon umfasst.

4. Das Verfahren zur Förderung des Blühens einer Pflanze nach irgendeinem der Ansprüche 1 bis 3, wobei das Florigen ein mutiertes Hd3a Protein, welches eine Aminosäuresequenz, die in SEQ ID NO: 1 angegeben ist, mit einer Substitution von 1 bis 10 Aminosäuren aufweist, oder ein Teilpeptid davon umfasst.

5. Das Verfahren zur Förderung des Blühens einer Pflanze nach irgendeinem der Ansprüche 1 bis 4, wobei das Florigen ausgewählt wird aus den folgenden mutierten Florigenen (1) bis (5):
(1) ein mutiertes Hd3a, das eine Aminosäuresequenz, die in SEQ ID NO: 1 angegeben ist, mit Substitutionen von Lysin an Position 31 und Glutaminsäure an Position 57 aufweist;
(2) ein mutiertes Hd3a, das eine Aminosäuresequenz, die in SEQ ID NO: 1 angegeben ist, mit Substitutionen von Cystein an Position 43, Cystein an Position 109, und Cystein an Position 166 aufweist;
(3) ein Teilpeptid des mutierten Hd3a nach (2), welches eine Aminosäuresequenz von Position 6 bis Position 170 der Aminosäuresequenz beinhaltet, die in SEQ ID NO: 1 angegeben ist;
(4) ein mutiertes Hd3a, das eine Aminosäuresequenz, die in SEQ ID NO: 1 angegeben ist, mit Substitutionen von Lysin an Position 31, Tyrosin an Position 136, und Tryptophan an Position 140 aufweist; und
(5) ein Teilpeptid des mutierten Hd3a nach (4), welches eine Aminosäuresequenz von Position 8 bis Position 170 der Aminosäuresequenz, die in SEQ ID NO: 1 angegeben ist, beinhaltet.

6. Das Verfahren zur Förderung des Blühens einer Pflanze nach irgendeinem der Ansprüche 1 bis 5, wobei ein Molverhältnis vom Florigen und dem Zell-penetrierenden Peptid in der gemischten Lösung 1:1 bis 1:50 beträgt.

7. Das Verfahren zur Förderung des Blühens einer Pflanze nach irgendeinem der Ansprüche 1 bis 6, wobei das wässrige Medium einen pH-Wert von 6.5 bis 8.0 aufweist.

8. Das Verfahren zur Förderung des Blühens einer Pflanze nach irgendeinem der Ansprüche 1 bis 7, wobei das wässrige Medium eine phosphatgepufferte Salzlösung (PBS) umfasst.

9. Eine Verwendung eines Mittels zur Förderung des Blühens einer Pflanze durch Einführen eines Florigens in eine Zelle eines Sprossapikalgewebes einer Pflanze, das Mittel umfassend:
ein Florigen; und
ein Zell-penetrierendes Peptid,
wobei das Mittel mit dem Sprossapikalgewebe der Pflanze in Kontakt gebracht wird.

10. Die Verwendung nach Anspruch 9, wobei das Mittel eine gemischte Lösung umfasst, die durch Mischen des Florigens und des Zell-penetrierenden Peptids in einem wässrigen Medium erhalten wird.

11. Verwendung eines Kits zur Förderung des Blühens einer Pflanze durch Einführen eines Florigens in eine Zelle eines Sprossapikalgewebes einer Pflanze, das Kit umfassend:
ein Florigen; und
ein Zell-penetrierendes Peptid,
wobei das Mittel mit dem Sprossapikalgewebe der Pflanze in einem Zustand, in dem das Florigen und das Zell-penetrierende Peptid koexistieren, in Kontakt gebracht wird.

## Revendications

1. Méthode pour promouvoir la floraison d'une plante comprenant l'utilisation d'une méthode d'introduction d'un florigène dans une cellule du tissu apical caulinaire d'une plante à l'aide d'un peptide de pénétration cellulaire, comprenant l'étape de mise en contact d'une solution mixte obtenue en mélangeant un florigène et un peptide de pénétration cellulaire dans un milieu aqueux avec le tissu apical caulinaire de la plante.

2. Méthode pour promouvoir la floraison d'une plante selon la revendication 1, dans laquelle le peptide de pénétration cellulaire est choisi parmi un peptide comprenant de 8 à 12 résidus arginine consécutifs et un peptide comportant une séquence RXLR.

3. Méthode pour promouvoir la floraison d'une plante selon la revendication 1 ou 2, dans laquelle le florigène comprend un florigène muté ayant une séquence d'acides aminés exposée dans SEQ ID No : 1 avec une substitution d'au moins un acide aminé choisi parmi les acides aminés correspondant aux résidus lysine à la position 31, cystéine à la position 43, acide glutamique à la position 57, cystéine à la position 109, tyrosine à la position 136, tryptophane à la position 140, et cystéine à la position 166, ou un peptide partiel de celui-ci.

4. Méthode pour promouvoir la floraison d'une plante selon l'une quelconque des revendications 1 à 3, dans laquelle le florigène comprend une protéine Hd3a mutée ayant une séquence d'acides aminés exposée dans SEQ ID No : 1 avec une substitution de 1 à 10 acides aminés, ou un peptide partiel de celle-ci.

5. Méthode pour promouvoir la floraison d'une plante selon l'une quelconque des revendications 1 à 4, dans laquelle le florigène est choisi parmi les florigènes mutés (1) à (5) suivants :
(1) une Hd3a mutée ayant une séquence d'acides aminés exposée dans SEQ ID No : 1 avec substitutions des résidus lysine à la position 31 et acide glutamique à la position 57 ;
(2) une Hd3a mutée ayant une séquence d'acides aminés exposée dans SEQ ID No : 1 avec substitutions des résidus cystéine à la position 43, cystéine à la position 109, et cystéine à la position 166 ;
(3) un peptide partiel de la Hd3a mutée décrite en (2), qui comprend une séquence d'acides aminés de la position 6 à la position 170 dans la séquence d'acides aminés exposée dans SEQ ID No : 1 ;
(4) une Hd3a mutée ayant une séquence d'acides aminés exposée dans SEQ ID No : 1 avec substitutions des résidus lysine à la position 31, tyrosine à la position 136, et tryptophane à la position 40 ; et
(5) un peptide partiel de la Hd3a mutée décrite en (4), qui comprend une séquence d'acides aminés de la position 8 à la position 170 dans la séquence d'acides aminés exposée dans SEQ ID No : 1.

6. Méthode pour promouvoir la floraison d'une plante selon l'une quelconque des revendications 1 à 5, dans laquelle un rapport molaire du florigène et du peptide de pénétration cellulaire dans la solution mixte est de 1/1 à 1/50.

7. Méthode pour promouvoir la floraison d'une plante selon l'une quelconque des revendications 1 à 6, dans laquelle le milieu aqueux a un pH de 6,5 à 8,0.

8. Méthode pour promouvoir la floraison d'une plante selon l'une quelconque des revendications 1 à 7, dans laquelle le milieu aqueux comprend une solution salée à tampon phosphate (PBS).

9. Utilisation d'un agent pour promouvoir la floraison d'une plante par introduction d'un florigène dans une cellule du tissu apical caulinaire d'une plante, l'agent comprenant :
un florigène ; et
un peptide de pénétration cellulaire,
dans laquelle l'agent est mis en contact avec le tissu apical caulinaire de la plante.

10. Utilisation selon la revendication 9, dans laquelle l'agent comprend une solution mixte obtenue en mélangeant le florigène et le peptide de pénétration cellulaire dans un milieu aqueux.

11. Utilisation d'un kit pour promouvoir la floraison d'une plante par introduction d'un florigène dans une cellule du tissu apical caulinaire d'une plante, le kit comprenant :
un florigène ; et
un peptide de pénétration cellulaire,
dans laquelle l'agent est mis en contact avec le tissu apical caulinaire de la plante dans un état où le florigène et le peptide de pénétration cellulaire coexistent.
